(19) Europäisches Patentamt
European Patent Office
Office européen des brevets

(11) **EP 1 705 476 B1**

(12) **EUROPEAN PATENT SPECIFICATION**

(45) Date of publication and mention
of the grant of the patent:
**19.02.2014 Bulletin 2014/08**

(51) Int Cl.:
***G01N 21/47*** *(2006.01)*   ***A61B 5/00*** *(2006.01)*

(21) Application number: **05027008.1**

(22) Date of filing: **09.12.2005**

(54) **Optical tomography apparatus**

Optisches Tomographiegerät

Appareil de tomographie optique

(84) Designated Contracting States:
**DE FR GB**

(30) Priority: **25.03.2005 JP 2005089993**
**06.06.2005 JP 2005165734**
**07.12.2005 JP 2005353158**

(43) Date of publication of application:
**27.09.2006 Bulletin 2006/39**

(73) Proprietor: **TOPCON CORPORATION**
**Tokyo 174-8580 (JP)**

(72) Inventors:
• **Toida, Masahiro**
**Ashigarakami-gun**
**Kanagawa-ken (JP)**
• **Tsujita, Kazuhiro**
**Ashigarakami-gun**
**Kanagawa-ken (JP)**
• **Morishima, Yoshikatsu**
**Ashigarakami-gun**
**Kanagawa-ken (JP)**
• **Fujita, Hiroshi**
**Saitama-shi**
**Saitama-ken (JP)**

(74) Representative: **Klunker . Schmitt-Nilson . Hirsch**
**Patentanwälte**
**Destouchesstrasse 68**
**80796 München (DE)**

(56) References cited:
**US-A- 5 329 134        US-A- 5 874 803**
**US-A1- 2002 196 446**

• SHIDLOVSKI V ET AL: "Superluminescent diodes for optical coherence tomography" PROCEEDINGS OF THE SPIE, SPIE, BELLINGHAM, VA, US, vol. 4648, 2002, pages 139-147, XP002372505 ISSN: 0277-786X
• BIZHEVA K K ET AL: "High resolution spectroscopic optical coherence tomography in the 900-1100 nm wavelength range" PROCEEDINGS OF THE SPIE, SPIE, BELLINGHAM, VA, US, vol. 4619, 2002, pages 249-252, XP002372506 ISSN: 0277-786X

EP 1 705 476 B1

**Description**

**Field of the Invention**

The present invention relates to an optical tomography apparatus that irradiates a low coherence measuring light beam onto a measurement target to obtain tomographic images of the measurement target. Particularly, the present invention relates to an optical tomography apparatus that obtains images of the surface and the fine structures within the measurement target, based on a reflected light beam, which is the measuring light beam reflected by the measurement target.

**Description of the Related Art**

As a conventional method for obtaining tomographic images of measurement targets, such as living tissue, a method that obtains optical tomographic images by SD-OCT (Spectral Domain Optical Coherence Tomography) measurement has been proposed (refer to U. S . Patent No. 6,377,349). The SD-OCT measurement is a type of light interference measurement method that utilizes the fact that light interference is detected only when the optical path lengths of divided light beams, that is, a measurement light beam and a reference light beam, match within a range of coherence length of a light source. That is, in this method, a low coherent light beam emitted from a light source is divided into a measuring light beam and a reference light beam, the measuring light beam is irradiated onto a measurement target, and the measurement light beam reflected by the measurement target is guided to a multiplexing means.

In an SD-OCT apparatus, the wide band low coherence light beam is divided into the measuring light beam and the reference light beam. The optical path lengths of the measuring light beam and the reference light beam are substantially matched, then the two light beams are caused to interfere with each other, to form a coherent light beam. Thereafter, the coherent light beam is decomposed into different frequency components by a spectral decomposing means. An array type photodetector measures the intensity of each frequency component of the coherent light beam. The coherent spectral waveform obtained by the photodetector undergoes Fourier transform at a computer, to obtain one dimensional tomographic data in the direction of the optical axis, without physically changing the optical path length. By scanning the measuring light beam in directions perpendicular to the optical axis, two dimensional and three dimensional tomographic images can be obtained.

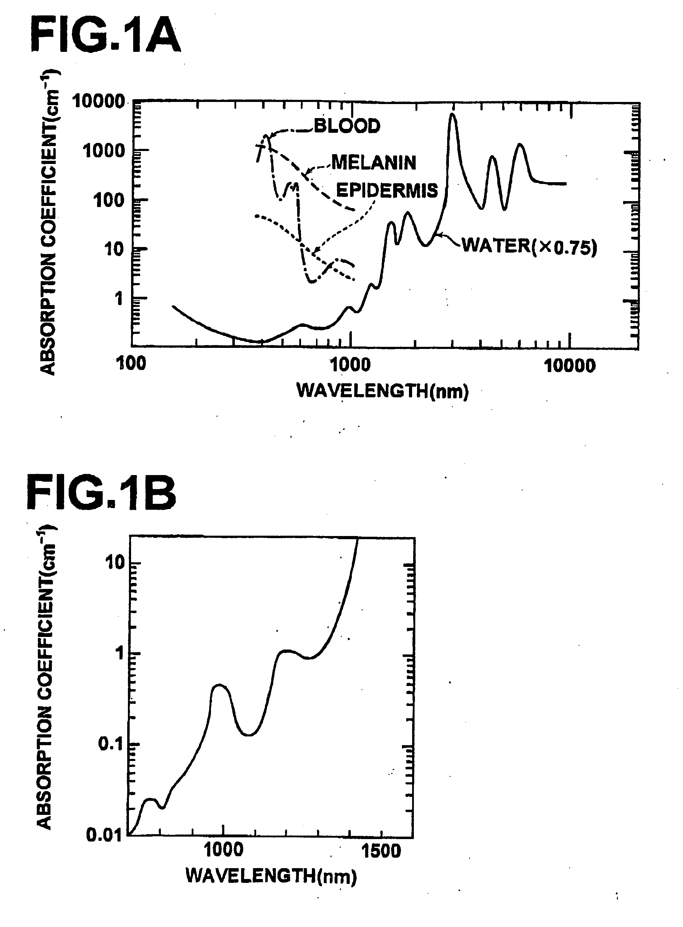

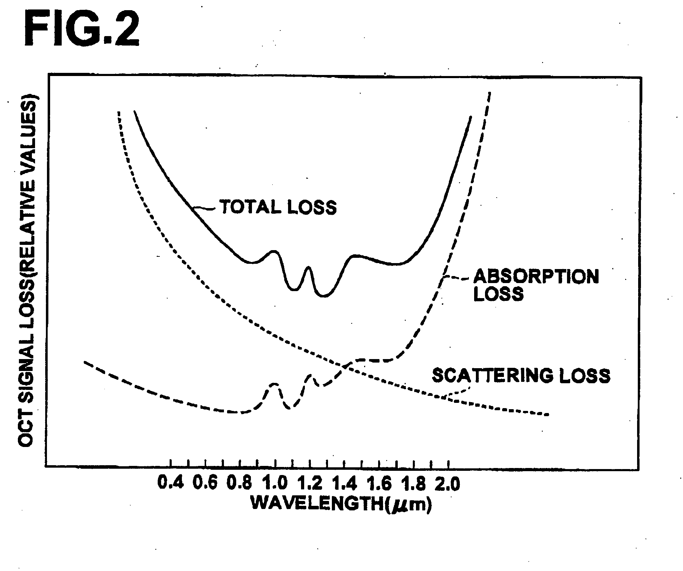

OCT apparatuses have been developed and are in use in the field of ophthalmology. Following the use of OCT apparatuses in the field of ophthalmology, research and development are underway for application in endoscopes. In the initial stages of development, the $0.8\mu m$ band had been employed as the wavelength of the light sources of the OCT apparatuses (refer to "In vivo ultrahigh-resolution optical coherence tomography", by W. Drexler et al., Optics Letters, Vol. 24, No. 17, pp. 1221-1223, 1999.). This wavelength band was selected as a result of considering absorption properties of living tissue. Figure 1A is a graph that illustrates light absorption coefficients of water, blood, melanin, and epidermis. Figure 1B is a graph that illustrates the absorption coefficients of water with respect to light having wavelengths between 0.7um and $1.6\mu m$. From the graph of Figure 1B, it can be seen that the peak of absorption occurs at 0.98 $\mu m$ and at $1.2\mu m$. In addition, the broken line in the graph of Figure 2 is a graph that represents absorption loss in living tissue, based on the absorption coefficients. From the graph of Figure 2, it can be seen that light within the $0.8\mu m$ band has the smallest amount of absorption loss. For this reason, it was considered that light within the $0.8\mu m$ band has the highest transmissivity with respect to living tissue, enables deeper measurement depths, and is most suited for OCT apparatuses.

However, it has been found recently that scattering properties also limit measurement depths in OCT apparatuses. This is because OCT apparatuses detect backscattered reflected light beams from within living tissue. Rayleigh scattering is common within living tissue. In Rayleigh scattering, the scattering intensity is inversely proportionate to wavelength to the fourth power. The dotted line in the graph of Figure 2 represents scattering loss within living tissue. The total loss, represented by the solid line in the graph of Figure 2, is the sum of the absorption loss and the scattering loss.

From the graph of Figure 2, it can be seen that the wavelength band, at which total loss is minimal, is the $1.3\mu m$ band. For this reason, after OCT apparatuses for ophthalmology were realized, research and development for OCT apparatuses to be applied to endoscopes, which require deeper imaging depths, are being performed with the $1.3\mu m$ band as the wavelength of light sources therein (refer to "Ultrahigh-resolution optical coherence tomography using continuum generation in an air-silica microstructure optical fiber", by I. Hartl et al., Optics Letters, Vol. 26, No. 9, pp. 608-610, 2001.).

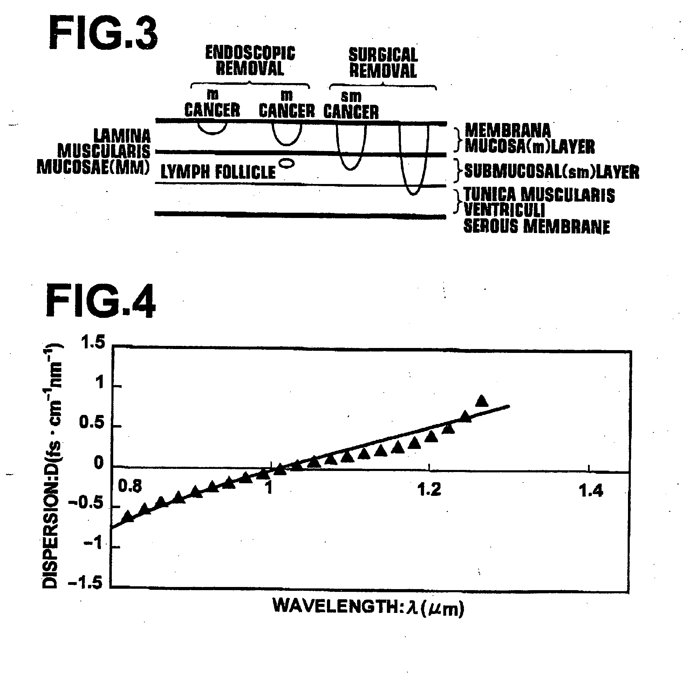

The purpose for applying an OCT apparatus to an endoscope is to enable definitive diagnoses within living organisms, and to diagnose the depth of tumor invasion of mucosal cancer (m cancer) and submucosal cancer (sm cancer). Hereinafter, the procedure of endoscopic diagnosis of cancer will be briefly described. First, a diseased portion is discovered within a normal observation image, and whether the disease is cancer or another illness is discriminated. This preliminary diagnosis is based on the experience of a physician, after which tissue from a portion estimated to be cancerous is collected and subjected to a biopsy, to obtain a definitive diagnosis. For this reason, it is presently difficult to obtain definitive diagnoses during examination with an endoscope. In the case that a diseased portion is definitively diagnosed as cancer, the depth of tumor invasion is diagnosed by endoscopic examination, in order to determine a treatment strategy. Commonly, cancers present themselves in the mucoepidermis, and metastasize in the horizontal direction and in the depth direction, as the disease progresses. As illustrated in Figure 3, the structure of a stomach wall is constituted by: a membrana mucosa (m) layer; lamina muscularis mucosae (MM) ; a submucosal (sm) layer; tunica muscularis ventriculi; and a serous membrane. Cancers which are present only in the membrana mucosa layer are designated as m cancers, and cancers which have penetrated to the submucosal layer are designated as sm cancers. Treatment protocols differ between m cancers and sm cancers. Blood vessels and lymph systems are present in the submucosal layer, and there is a possibility of metastasis in the case of sm cancers. Therefore, surgical procedures are required. On the other hand, there is no possibility of metastasis in the case of m cancers. Therefore, m cancers are removed by endoscopic procedures. For this reason, it is necessary to discriminate whether cancers are m cancers or sm cancers. Specifically, it is important to be able to evaluate whether the layer structure of the lamina muscularis mucosae layer is maintained or destroyed, in an image. Presently, application of ultrasound imaging techniques is being considered, with the objective of diagnosing the depth of tumor invasion. However, the resolution of ultrasound imaging is only about 100$\mu$m in the axial direction, which is insufficient to visualize the MM layer. In addition, in m cancers which have progressed, lymph follicles are formed under the MM layer, thereby causing the cancerous portions and the lymph follicles to be imaged integrally, and m cancers may be misdiagnosed as sm cancers. For this reason, an imaging method having a resolution of 10$\mu$m or less in the axial direction is desired, to enable accurate diagnosis of the depth of tumor invasion.

Meanwhile, the resolution of an OCT apparatus in the optical axis direction is determined by the coherence length of the light source. That is, it is not generally possible to obtain resolution less than the coherence length of the light source. For this reason, a light beam having a coherence length of 10$\mu$m or less is necessary to obtain high resolution of 10$\mu$m or less. The coherence length $\Delta z$ of low coherence light is proportionate to the square of the central frequency and inversely proportionate to the spectrum width thereof. The coherence length $\Delta z$ can be expressed by the following formula:

$$\Delta z = (2\ln 2/\Pi) \cdot (\lambda c^2/\Delta\lambda)$$

wherein

$\lambda$c: central wavelength
$\Delta\lambda$: spectrum width

For this reason, it is necessary to broaden the spectrum width $\Delta\lambda$ in order to decrease the coherence length. Meanwhile, it was found that the influence of dispersion needed to be considered, if the spectrum width $\Delta\lambda$ was broadened (refer to "Optimal wavelength for ultrahigh-resolution optical coherence tomography", by Y. Wang et al., Optics Express, Vol. 11, No. 12, pp. 1411-1417, 2003.).

In a Michaelson interferometer, as a light beam propagates through a sample, phase shift occurs, and a coherent signal waveform changes as a result. If the coherent signal waveform is designated as $\varphi(w)$ and the spectrum waveform of the light source is a Gaussian distribution, autocorrelation functions can be expressed as:

$$\delta_t = \delta_{t0} \cdot \left\{ 1 + \left\langle \frac{d^2\varphi(w)}{dw^2} \right\rangle \delta w^4 \right\}^{\frac{1}{2}} \tag{1}$$

$$K = \delta_t / \delta_{t0} \tag{2}$$

$$D = \frac{-w_0^2}{2\pi c} \cdot \frac{d^2\varphi(w)}{dw^2} \tag{3}$$

wherein

$\delta_t$: $1/e^{1/2}$ width of the autocorrelation function
$\delta_{to}$: $1/e^{1/2}$ width of the autocorrelation function when D=0
$\delta_w$: $1/e^{1/2}$ width of the optical spectrum
$W_0$: central frequency of the optical spectrum
K: broadening ratio due to the influence of dispersion

Figure 4 is a graph that illustrates calculated results (represented by the solid line) of formula (3) above and actual measured values (represented by the triangles) . Dispersion D is zero when the wavelength of the light beam is $1.0\mu m$. It can be seen from the graph of Figure 4 that the influence of dispersion becomes greater as the wavelength becomes greater than or less than $1.0\mu m$.

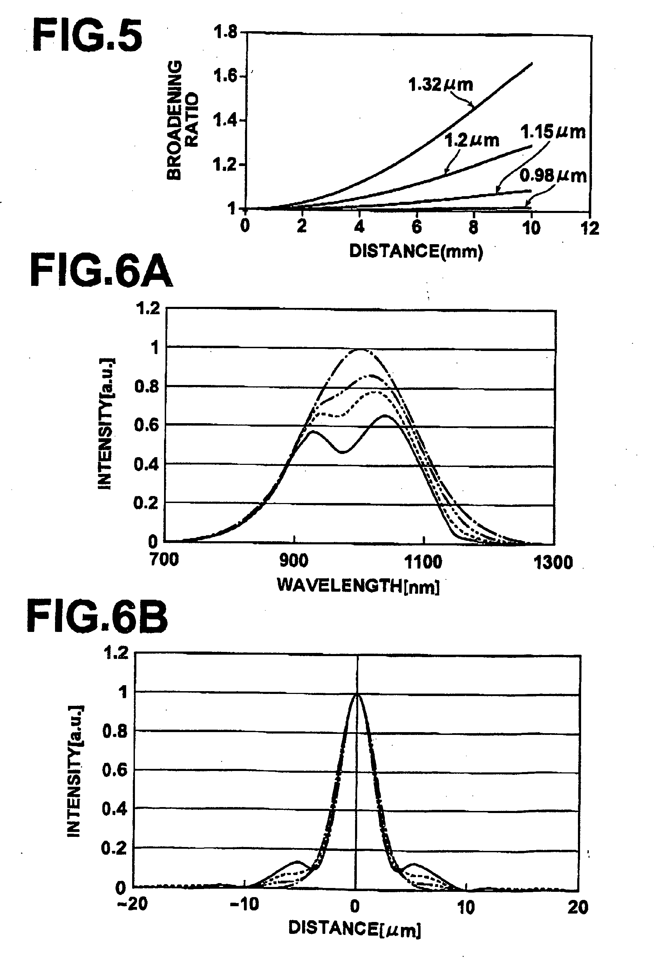

Figure 5 is a graph that illustrates simulation results of the relationship between the distance of propagation (depth of water) and broadening ratios, when low coherence light beams having wavelengths of $1.32\mu m$ (spectrum width: 75nm), $1.2\mu m$ (spectrum width 62nm), $1.15\mu m$ (spectrum width: 59nm), and $0.98\mu m$ (spectrum width: 41nm) propagate through water.

In the aforementioned document, Y. Wang et al. conclude that it is preferable to employ low coherence light having a central wavelength of $1.0\mu m$ in OCT apparatuses, in the case that the coherence length of the low coherence light beam is short.

However, when an OCT apparatus that employs low coherence light is used to obtain an optical tomographic image of an organism, there are cases in which the wavelength band of the low coherence light (measuring light beam) includes wavelengths which are readily absorbed by living tissue. In these cases, the spectral shape of the low coherence light (reflected light beam) changes due to the light absorption by the living tissue, and side bands or side lobes appear in the autocorrelation function, generating pseudo signals that reduce the S/N ratio of the optical tomographic image. As illustrated in Figure 1B, peaks in the absorption coefficient of water, which is the main constituent of living tissue, occur at wavelengths of $0.98\mu m$ and $1.2\mu m$.

Figures 6A and 6B are graphs that represent simulations of a light beam having a central wavelength of $1.0\mu m$, a wavelength band width of 100nm, and a Gaussian distribution propagating through water. Figure 6A represents the changes in spectrum shape, and Figure 6B represents Fourier transform waveforms of each spectral waveform. Note that the solid lines represent waveforms which are not transmitted through water; the long/short dashed lines represent waveforms which have been transmitted through 2mm of water; the long/short/short dashed lines represent waveforms which have been transmitted through 4mm of water; and the broken lines represent waveforms which have been transmitted through 8mm of water. It can be seen from the graphs of Figures 6A and 6B that in the case that a light beam having a central wavelength of $1\mu m$ and a wavelength band width of 100nm propagates through water, influence of absorption at $0.98\mu m$ greatly changes the spectrum shape. As a result, side bands appear in the autocorrelation waveform, pseudo signals are generated, and the quality of the optical tomographic image deteriorates.

In the aforementioned document by Y. Wang et al., it is disclosed that influence due to scattering is observed when optical tomographic images are obtained employing low coherence light having a coherence length of approximately $10\mu m$ ($\lambda c^2/\Delta\lambda$=23) . In addition, Y. Wang et al. disclose that it is preferable to set the central wavelength of low coherence light in the vicinity of $1.0\mu m$ in cases that influence due to dispersion is observed. However, there is no disclosure regarding a central wavelength $\lambda c$ nor a wavelength band width AX that avoids influence due to absorption at the $0.98\mu m$ and $1.2\mu m$ wavelengths.

In accordance with the preamble of claim 1, SHIDLOVSKI V ET AL: "Superlumin-escent diodes for optical coherence tomography" PROCEEDINGS OF THE SPIE - THE INTERNATIONAL SOCIETY FOR OPTICAL ENGINEERING SPIE-INT. SOC. OPT. ENG USA, vol. 4648. 2002, pages 139-147, XP002372505 ISSN: 0277-786X discloses an optical tomography apparatus including a light source in form of a superluminescent diode (SLED). The specific nature of this device is not disclosed in the reference.

SUMMARY OF THE INVENTION

The present invention has been developed in view of the aforementioned problems. It is an object of the present invention to clarify the presence of optimal wavelength properties for obtaining high resolution while taking into consideration the light absorption properties, the scattering properties, and the dispersion properties of living organisms. It is another object of the present invention to realize an optical tomography apparatus that employs low coherence light having the optimal wavelength properties to obtain high resolution optical tomographic images having high image quality.

The optical tomography apparatus of the present invention comprises the features of claim 1.

The central wavelength $\lambda c$ and the full width at half maximum spectrum $\Delta\lambda$ of the reference light beam and the reflected light beam may satisfy the following condition:

$$\lambda c^2/\Delta\lambda \leq 17.$$

Note that here, the "reflected light beam" refers to: light which is reflected by the measurement target; light which is backscattered by the measurement target; and light which is both reflected and backscattered by the measurement target.

In addition, the reference light beam and the reflected light beam have substantially the same central wavelengths and full width at half maximum spectra. The phrase "substantially the same" refers to a degree of uniformity that does not cause adverse effects in measurement of the intensity of the coherent light beam.

The image obtaining means may detect the intensity for each frequency of the coherent light beam; and calculate the intensity of the reflected light beam at the plurality of depth positions of the measurement target, based on the detected intensities for each frequency of the coherent light beam.

The light source comprises a super luminescent diode. The super luminescent diode comprises: a GaAs substrate having a first conductivity; an optical waveguide path constituted by an InGaAs active layer on the GaAs substrate; and a window region layer having a greater energy gap and a smaller refractive index than the active layer and a second conductivity different from the first conductivity, constituted by a binary or ternary semiconductor material with a lattice coefficient that lattice matches with GaAs within a range of $\pm 0.1\%$ and does not contain Al, provided at a rear emitting facet of the optical waveguide path. The semiconductor material of the window region layer is one of GaAs and InGaP.

The light source may comprise phosphor that contains near infrared fluorescent pigment.

The light source may comprise one of a Yb type pulse laser, an Nd type pulse laser, and a Ti type pulse laser. Note that a Yb:YAG laser, a Yb:Glass laser, or a Yb type fiber laser may be utilized as the Yb type pulse laser. An Nd:YAG laser, an Nd:Glass laser, or an ND type fiber laser may be utilized as the Nd type pulse laser.

Further, the optical tomography apparatus may comprise a Gaussian spectrum forming filter, in addition to the aforementioned structures. In this case, the central wavelength and the full width at half maximum spectrum of the low coherence light beam emitted from the light source itself may be of any values, as long as the low coherence light beam satisfies the above conditions after passing through the Gaussian spectrum forming filter.

The optical tomography apparatus of the present invention comprises: a light source, for emitting low coherence light beam having; dividing means, for dividing the low coherence light beam into a measuring light beam and a reference light beam; an irradiating optical system, for irradiating the measuring light beam onto a measurement target; multiplexing means, for multiplexing a reflected light beam, which is the measuring light beam reflected by the measurement target, and the reference light beam, to obtain a coherent light beam; and image obtaining means, for calculating the intensity of the reflected light beam at a plurality of depth positions of the measurement target, based on the properties of the multiplexed coherent light beam, and for obtaining tomographic images of the measurement target, based on the intensities at each of the depth positions; a central wavelength $\lambda c$ and a full width at half maximum spectrum $\Delta\lambda$ of the reference light beam and the reflected light beam satisfying the conditions: $\lambda\ c^2/\Delta\lambda \leq 23$; $\lambda c+(\Delta\lambda/2) \leq 1.2\mu m$; and $\lambda c-(\Delta\lambda/2) \geq 0.98\mu m$. Therefore, the transmissivity of the light beam is favorable, and the influence of light absorption having its peaks at the wavelengths $0.98\mu m$ and $1.2\mu m$ is reduced. Accordingly, high resolution optical tomographic images having high image quality can be obtained. In the case that the value of $\lambda c^2/\Delta\lambda$ is large, that is, the coherence length is long and the full width at half maximum spectrum is narrow, there is almost no influence due to dispersion by water. However, when the value of $\lambda c^2/\Delta\lambda$ is small, the influence due to dispersion by water cannot be ignored.

From the simulation results of Figure 5, it can be seen that if the central wavelength is in the vicinity of $1.3\mu m$, influence due to dispersion is observed even if the spectrum width is 75nm, that is, the coherence length is approximately $10\mu m$ ($\lambda c^2/\Delta\lambda \approx 23$).

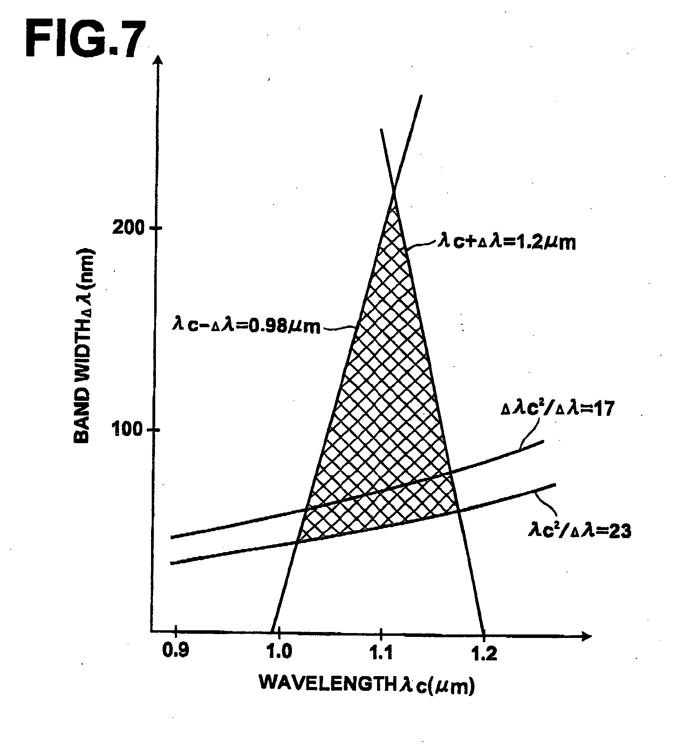

That is, it is considered that a light beam having a central wavelength of $1.0\mu m$ is superior to that having a central wavelength of $1.3\mu m$, if the value of $\lambda c^2/\Delta\lambda$ is less than or equal to 23. Note that Figure 7 is a graph that represents central wavelengths $\lambda c$ and full width at half maximum spectra $\Delta\lambda$ that satisfy the above conditions.

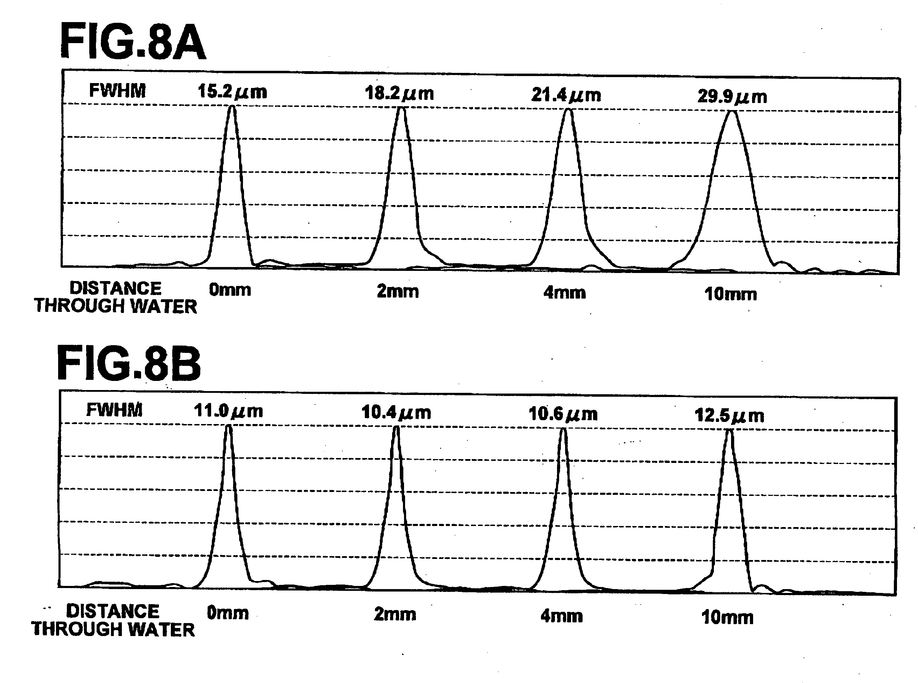

The inventor caused a low coherence light beam having a central wavelength ($\lambda c$) of $1.32\mu m$ and full width at half maximum spectrum ($\Delta\lambda$) of 100nm to be transmitted through water for distances of 2mm, 4mm, and 10mm, to observe deterioration in resolution along the optical axis direction. The results of the observations are graphed in Figure 8A. At the same time, a low coherence light beam having a central wavelength ($\lambda c$) of $1.15\mu m$ and full width at half maximum spectrum ($\Delta\lambda$) of 100nm to be transmitted through water for distances of 2mm, 4mm, and 10mm, to observe deterioration in resolution along the optical axis direction. The results of the observations are graphed in Figure 8B. With a spectrum width of 100nm, broadening of the resolution in the optical axis direction was conspicuous for the low coherence light beam having the central wavelength of $1.32\mu m$. In contrast, it can be seen from Figure 8B that very little deterioration in the resolution in the optical axis direction occurred for the low coherence light beam having the central wavelength of $1.15\mu m$. From these measurement results, the range, in which light beams having central wavelengths in the $1.0\mu m$ band are superior to those having central wavelengths in the $1.3\mu m$ band can be calculated as

$\lambda C^2/\Delta\lambda\backslash=1.32^2/0.1\approx17$. From this, the range can be defined as:

$$\lambda c^2/\Delta\lambda \leq 17.$$

The image obtaining means may detect the intensity for each frequency of the coherent light beam; and calculate the intensity of the reflected light beam at the plurality of depth positions of the measurement target, based on the detected intensities for each frequency of the coherent light beam. In this case, intensities of the reflected light beam at the plurality of depth positions of the measurement target can be calculated with a single irradiation of the measuring light beam. Accordingly, tomographic images of the measurement target can be obtained within a short period of time.

By employing a super luminescent diode as the light source for the optical tomography apparatus, a low-cost and easily handled apparatus can be realized. The present inventors focused on a super luminescent diode having a window structure at an end facet of an optical waveguide path as the light source for the optical tomography apparatus. Development of the super luminescent diode was initiated, but desirable element life was not obtainable with conventionally known structures for super luminescent diodes. Through experimentation, the present inventors discovered that there are important conditions other than the material of the window region layer that need to be considered.

First, consider a case that an InGaAs active layer is employed to obtain a light beam having a central wavelength greater than or equal to $0.98\mu m$ and less than $1.2\mu m$. InGaAs deteriorates at temperatures of 650° and greater. Therefore, it is preferable that processing steps following forming of the active layer be performed within an atmosphere of 650° or less. AlGaAs is frequently utilized as the material for windows, in super luminescent diodes having window structures as described above. However, it is known that it is preferable to form semiconductor layers containing Al at high temperatures. This is because oxygen is incorporated into the material when the atmospheric temperature is low (refer to F. Bugge et al., J. Crystal Growth Vol. 272 (2004) pp. 531-537).

In experiments, window region layers were produced with materials that contain Al, for example, AlGaAs, at temperatures less than or equal to 650°. In these cases, oxygen was incorporated into the window region layer, thereby increasing non-light emitting recombined centers. The increased non-light emitting recombined centers generate heat, thereby causing it to be difficult to obtain a desired element life.

Generally, active layers are approximately 100Å thick, whereas it is necessary to grow films at least several hundreds of nm, to form window region layers. In experiments, films for window region layers were formed on a GaAs substrate, from materials that do not lattice match with GaAs. It was learned that in these cases, the crystal film quality deteriorated, thereby causing it to be difficult to obtain a desired element life. For example, consider a case in which InGaAs, which has less In than InGaAs that forms the active layer, is employed to form the window region layer. In this case, the InGaAs does not lattice match with the GaAs substrate. Therefore, an InGahs layer having favorable crystal film quality cannot be formed, thereby causing it to be difficult to obtain a desired element life.

InGaAsP is a material that lattice matches with the GaAs substrate, and has a greater energy gap than the InGaAs active layer. However, it was learned in experiments that if such quaternary semiconductor materials are employed as the material for a layer, such as a window region layer, which is stacked on various crystal surfaces, the ratio of the four materials becomes unbalanced, deteriorating the crystal film quality. In addition, if the crystal is grown at atmospheric temperatures less than or equal to 650°, hillocks are generated and crystal film quality deteriorates, thereby decreasing the reliability of the element.

Accordingly, a super luminescent diode comprising: a GaAs substrate having a first conductivity; an optical waveguide path constituted by an InGaAs active layer on the GaAs substrate; and a window region layer having a greater energy gap and a smaller refractive index than the active layer and a second conductivity different from the first conductivity, constituted by a binary or ternary semiconductor material with a lattice coefficient that lattice matches with GaAs within a range of $\pm0,1\%$ and does not contain Al, provided at a rear emitting facet of the optical waveguide path, is employed. The binary or ternary semiconductor material is one of GaAs and InGaP. By employing such a super luminescent diode, a low coherence light beam having an undistorted sectional beam shape can be emitted easily and at low cost. In addition, by employing a light source comprising such a super luminescent diode, the reliability of the light source is improved, and as a result, the reliability of the optical tomography apparatus is also improved.

The light source may comprise phosphor that contains near infrared fluorescent pigment. In this case, a low coherence light beam having a desired wavelength band can be employed.

The light source may comprise one of a Yb type pulse laser, an Nd type pulse laser, and a Ti type pulse laser. In this case, a high output low coherence light beam can easily be employed.

Further, the light source may comprise a Gaussian spectrum forming filter. In this case, the low coherence light beam emitted from the light source itself may have any central wavelength and any full width at half maximum spectrum, thereby increasing the options for the light source. In addition, the spectrum shape of the low coherence light beam will be of a Gaussian distribution, and higher quality optical tomographic images can be obtained.

## BRIEF DESCRIPTION OF THE DRAWINGS

Figure 1A is a graph that illustrates light absorption coefficients of water, blood, melanin, and epidermis.

Figure 1B is a graph that illustrates the absorption coefficients of water with respect to light having wavelengths between $0.7\mu m$ and $1.6\mu m$.

Figure 2 is a graph for explaining absorption loss in living tissue, based on absorption coefficients.

Figure 3 is a diagram for explaining the progression of cancer in a stomach wall.

Figure 4 is a graph for explaining dispersion properties of water.

Figure 5 is a graph for explaining the relationship between distances of propagation in water and broadening ratios.

Figures 6A and 6B are graphs for explaining spectral waveforms and Fourier transforms thereof, respectively.

Figure 7 is a graph for explaining the relationship between central wavelengths and spectral bandwidths.

Figures 8A and 8B are graphs for explaining the relationship between distance propagated through water and resolution in the optical axis direction.

Figure 9 is a schematic diagram that illustrates the construction of an optical tomography apparatus according to an embodiment of the present invention.

Figure 10 is a schematic diagram that illustrates the construction of an SLD.

Figure 11 is a schematic diagram that illustrates the construction of an alternate SLD.

Figure 12 is a schematic diagram that illustrates the construction of a first alternate light source unit.

Figure 13 is a schematic diagram that illustrates the construction of a second alternate light source unit.

Figure 14 is a graph for explaining absorption spectra of pigments.

Figure 15 is a graph for explaining fluorescence.

Figure 16 illustrates examples of pyrylium type pigment.

Figure 17 is a schematic diagram that illustrates the construction of a third alternate light source unit.

Figure 18 is a schematic diagram that illustrates a modified optical tomography apparatus.

## DESCRIPTION OF THE PREFERRED EMBODIMENTS

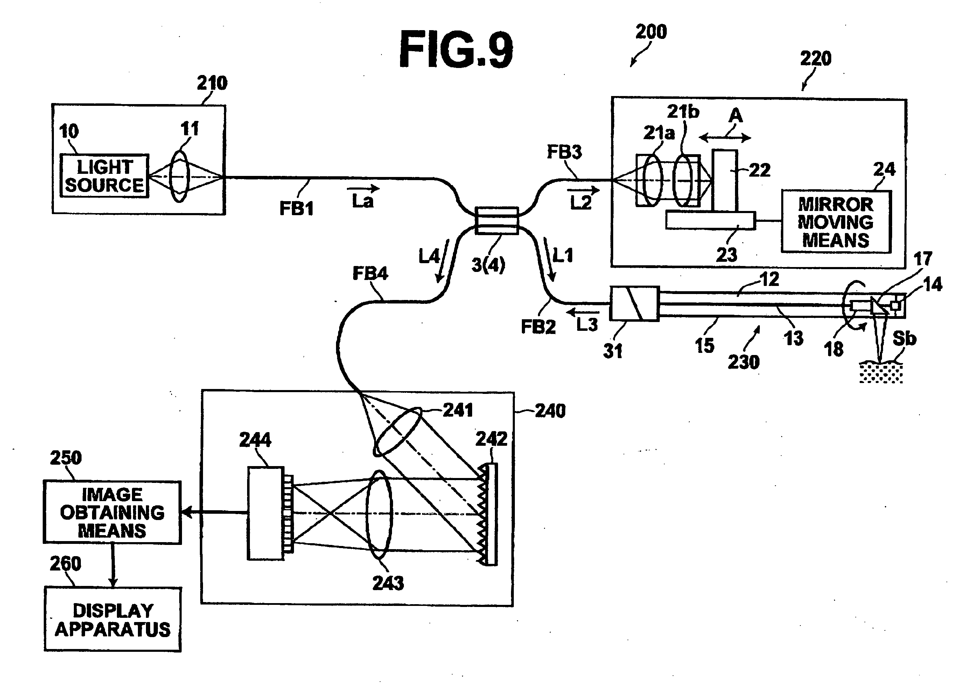

Hereinafter, an optical tomography apparatus 200 according to a first embodiment of the present invention will be described with reference to Figure 9. Figure 9 is a schematic diagram that illustrates the construction of the optical tomography apparatus 200.

The optical tomography apparatus 200 illustrated in Figure 9 obtains tomographic images of measurement targets, such as the interiors of body cavities of living organisms or cells, by the aforementioned SD-OCT measurement technique. The optical tomography apparatus 200 comprises: a light source unit 210, for emitting a low coherence light beam La; a light dividing means 3, for dividing the low coherence light beam La, which is emitted from the light source unit 210, into a measuring light beam L1 and a reference light beam L2; an optical path length adjusting means 220, for adjusting the optical path length of the reference light beam L2; an optical probe 230.that irradiates the measuring light beam L1 onto a measurement target S; a multiplexing means 4, for multiplexing a reflected light beam L3, which is the measuring light beam L1 reflected from the measurement target S, and the reference light beam L2; and a coherent light detectingmeans 240, for detecting a coherent light beam L4, formed by multiplexing the reflected light beam L3 and the reference light beam L2.

The light source unit 210 comprises: an SLD (Super Luminescent Diode) that emits a low coherence light beam La having a central wavelength $\lambda c$ of $1.1\mu m$ and a full width at half maximum spectrum $\Delta\lambda$ of 75nm as the light source 10; and the condensing lens 11, for causing the light beam La emitted from the light source 10 to enter the optical fiber FB1 as an optical system. Note that the detailed construction of the SLD will be described later.

The light dividing means 3 is constituted by a 2x2 optical fiber coupler, for example. The light dividing means 3 functions to divide the light beam La emitted by the light source unit 210 and guided through an optical fiber FB1 into the measuring light beam L1 and the reference light beam L2. The light dividing means 3 is optically connected to optical fibers FB2 and FB3. The measuring light beam L1 is guided through the optical fiber FB2, and the reference light beam L2 is guided through the optical fiber FB3. Note that the light dividing means 3 of the present embodiment also functions as the multiplexing means 4.

The optical fiber FB2 is optically connected to the optical probe 230, and the measuring light beam is guided through the optical fiber FB2 to the optical probe 230. The optical probe 230 is to be inserted into body cavities via a forceps opening and a forceps channel, and is removably mounted to the optical fiber FB2 with an optical connector 31.

The optical probe 230 comprises: a probe outer cylinder 15, which has a closed distal end; a single optical fiber 13, which is provided to extend along the axial direction of the outer cylinder 15 within the interior space thereof; a prism mirror 17, for deflecting a light beam L emitted from the distal end of the optical fiber 15; a rod lens 18, for condensing the light beam L such that it converges on the measurement target S, which surrounds the outer cylinder 15; and a motor 14, for rotating the prism mirror 17 with the axis of the optical fiber 13 as the rotational axis.

The optical path length adjusting means 220 is provided at the reference light beam L2 emitting side of the optical fiber FB3. The optical path length adjusting means 220 adjusts the optical path length of the reference light beam L2 to adjust the position at which obtainment of tomographic images is initiated. The optical path length adjusting means 220 comprises: a mirror 22, for reflecting the reference light beam L2 emitted from the optical fiber FB3; a first optical lens 21a, which is provided between the mirror 22 and the optical fiber FB3; and a second optical lens 21b, which is provided between the first optical lens 21a and the mirror 22.

The first optical lens 21a functions to collimates the reference light beam L2, which is emitted from the core of the optical fiber FB3, and also functions to focus the reference light beam L2, which is reflected by the mirror 22, onto the core of the optical fiber FB3. The second optical lens 21b functions to focus the reference light beam L2, which is collimated by the first optical lens 21a, onto the mirror 22, and also functions to collimate the reference light beam L2, which is reflected by the mirror 22. That is, the first optical lens 21a and the second optical lens 21b form a confocal optical system.

Accordingly, the reference light beam L2 emitted from the optical fiber FB3 is collimated by the first optical lens 21a, and focused onto the mirror 22 by the second optical lens 21b. Thereafter, the reference light beam L2 reflected by the mirror 22 is collimated by the second optical lens 21b, and focused onto the core of the optical fiber FB3 by the first optical lens 21a.

The optical path length adjusting means 220 further comprises: a base 23, on which the second optical lens 21b and the mirror 22 are fixed; and a mirror moving means 24, for moving the base 23 in the direction of the optical axis of the first optical lens 21a. The optical path length of the reference light beam L2 is varied by moving the base 23 in the directions indicated by arrow A.

The multiplexing means 4 is constituted by the aforementioned 2x2 optical coupler. The multiplexing means 4 multiplexes the reference light beam L2, of which the optical path length has been adjusted by the optical path length adjusting means 220, and the reflected light beam L3 reflected by the measurement target S. The multiplexed coherent light beam L4 is emitted toward the coherent light detecting means 240 via the optical fiber FB4.

The coherent light detecting means 240 detects the intensity of the coherent light beam L4. The coherent light detecting means 240 comprises: a collimating lens 241, for collimating the coherent light beam L4 emitted from the optical fiber FB4; a spectral decomposing means 242, for decomposing the coherent light beam L4 into each of its constituent wavelength bands; and a photodetecting means 244, for detecting each wavelength band of the coherent light beam L4.

The spectral decomposing means 244 is constituted by a diffraction grating element or the like. The spectral decomposing means 244 decomposes the coherent light beam L4 incident thereon, and emits the decomposed components toward the photodetecting means 244. The photodetecting means 244 is constituted by CCD elements or the like, in which optical sensors are arranged one dimensionally or two dimensionally. Each of the optical sensors are configured to detect each wavelength band component of the spectrally decomposed coherent light beam L4, respectively.

The photodetecting means 244 is connected to an image obtaining means 250, constituted by a computer system such as a personal computer. The image obtaining means 250 is connected to a display apparatus 260, constituted by a CRT display or an LCD.

Hereinafter, the operation of the optical tomography apparatus 200 of the above construction will be described. When obtaining a tomographic image, first, the base 23 is moved in the direction of arrow A, to adjust the optical path length such that the measurement target S is positioned within a measurable region. Thereafter, the light beam La is emitted from the light source unit 210. The light beam La is divided into the measuring light beam L1 and the reference light beam L2 by the light dividing means 3. The measuring light beam L1 is emitted within the body cavity from the optical probe 230, and irradiated on the measurement target S. At this time, the measuring light beam L1 is scanned one dimensionally along the measurement target S by the aforementioned operation of the optical probe 230. The reflected light beam L3, which is reflected by the measurement target S, is multiplexed by the reference light beam L2, which is reflected by the mirror 22, to form the coherent light beam L4. The coherent light beam L4 is detected by the coherent light detecting means 240. The detected coherent light beam L4 undergoes appropriate waveform correction and noise removal at the image obtaining means 250. Then, the coherent light beam L4 undergoes Fourier transform, and reflected light intensity distribution data is obtained regarding the depth direction of the measurement target S.

Next, the motor 14 of the optical probe 230 rotates the prism mirror 17, thereby scanning the measuring light beam L1 on the measurement target S. Thereby, data in the depth direction along the scanning direction can be obtained, and a tomographic image of tomographic sections that include the scanning direction can be obtained. The tomographic image obtained in this manner is displayed at a display apparatus 260. Note that by moving the optical probe 230 in the horizontal direction in Figure 9, the measuring light beam L1 can be scanned in a second direction perpendicular to the aforementioned scanning direction. Thereby, a tomographic image of tomographic sections that include the second direction can be further obtained.

In the case that a light beam having a central wavelength $\lambda$ c of 1.1$\mu$m and a full width at half maximum spectrum $\Delta\lambda$ is employed as the low coherence light beam La, $\lambda c^2/\Delta\lambda$ becomes 16.1. Therefore, if the effects of dispersion are taken into consideration, a central wavelength band of 1.0$\mu$m is superior to a central wavelength band of 1.3$\mu$m. In addition, in this case, the central wavelength $\lambda c$ and the full width at half maximum spectrum $\Delta\lambda$ satisfies the conditions:

$$\lambda c + (\Delta\lambda / 2) \leq 1.2\mu m$$

$$\lambda c - (\Delta\lambda / 2) \geq 0.98\mu m.$$

Therefore, the low measuring light beam L1 has good transmissivity with respect to the measurement target S, and the influence exerted on the reflected light beam L3 by the light absorption peaks of water at the wavelengths of 0.98$\mu$m and 1.2$\mu$m is decreased. Accordingly, high resolution optical tomographic images having high image quality can be obtained.

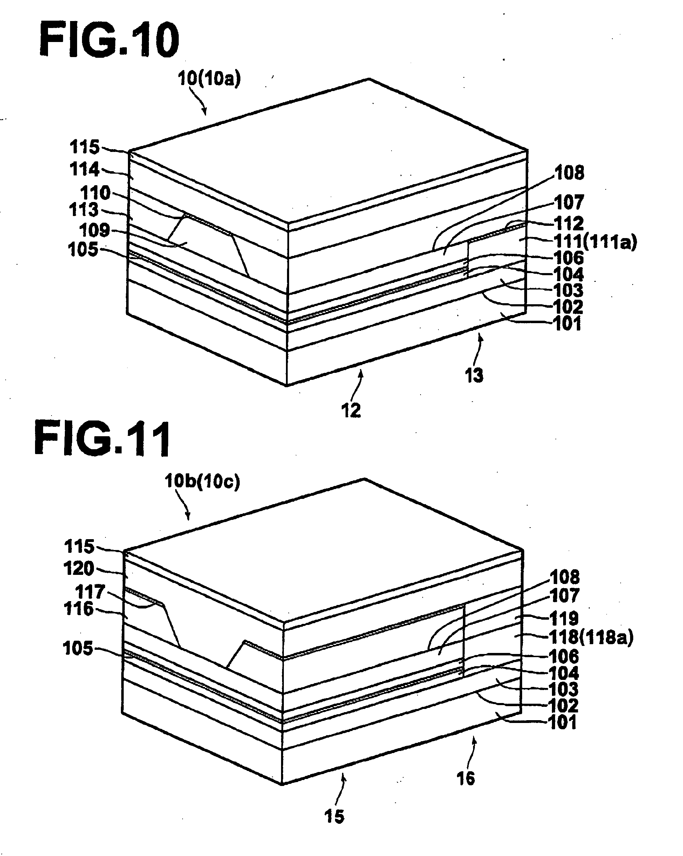

Next, the SLD 10 of the light source unit 210 will be described with reference to Figure 10. The SLD 10 is a super luminescent diode having an SBR structure formed by crystal growth using the metal organic chemical vapor deposition method (MOCVD). Figure 10 is a sectional view of the SLD 10. TEG (tri ethyl gallium), TMA (tri methyl aluminum), TMI (tri methyl indium), $AsH_3$ (arsine), $PH_3$ (phosphine), and the like are used as the source gas in the MOCVD method. $SiH_4$ (silane) and DEZ (di ethyl zinc) are employed as dopants.

The SLD 10 comprises: an optical waveguide path section 12; and a window section 13, which is provided at an end opposite a light emitting end of the optical waveguide path section 12. The optical waveguide path section 12 has an SBR structure comprising: a p-type GaAs etching stop layer 108; and a ridge-shaped p-type $In_{0.49}Ga_{0.51}P$ second upper cladding layer 109 formed on the etching stop layer 108. The second upper cladding layer 109 functions as an optical guide.

The SLD is formed by an n-type GaAs substrate 101, on which an n-type GaAs buffer layer 102 and an n-type $In_{0.49}Ga_{0.51}P$ cladding layer 103 are stacked in this order. The optical waveguide path section 12 is formed by: a non-doped GaAs lower optical guiding layer 104; an InGaAs multiple quantum well active layer 105; a non-doped GaAs upper optical guiding layer 106; a p-type $In_{0.49}Ga_{0.51}P$ first upper cladding layer 107, and the GaAs etching stop layer 108, which are stacked on the lower cladding layer 103 in this order. Note that an $In_xGa_{1-x}As$ composition having a ratio In:X>0.3 is employed as the material of the InGaAs multiple quantum well active layer 105.

The ridge-shaped p-type $In_{0.99}Ga_{0.51}P$ second upper cladding layer 109 is formed on the p-type GaAs etching stop layer 108. An n-$In_{0.49}(Al_{0.12}Ga_{0.88})_{0.51}P$ current blocking layer 113 is formed on the sides of the ridge (the p-type $In_{0.49}Ga_{0.51}P$ second upper cladding layer 109). A p-type GaAs cap layer 110 (0.1$\mu$m thick, with a carrier density of $7.0 \times 10^{17} cm^{-3}$); a p-type $In_{0.49}(Al_{0.12}Ga_{0.88})_{0.51}P$ third upper cladding layer 114; and a p-GaAs contact layer 115 are formed on the upper surfaces of the ridge and the n-$In_{0.49}(Al_{0.12}Ga_{0.88})_{0.51}P$ current blocking layer 113.

The window section 13 is formed by a p-type GaAs window region layer 111; an $In_{0.49}Ga_{0.51}P$ window region layer etching stop layer 112; the n-$In_{0.49}(Al_{0.12}Ga_{0.88})_{0.51}P$ current blocking layer 113; the p-type $In_{0.49}(Al_{0.12}Ga_{0.88})_{0.51}P$ third upper cladding layer 114; and the p-GaAs contact layer 115, which are stacked in this order on the n-type $In_{0.49}Ga_{0.51}P$ lower cladding layer.

In the SLD 10 having the construction described above, light, which is guided through the InGaAs multiple quantum well active layer 105 toward the window section 13, is emitted within the window region layer 111 and scattered. Thereby, laser oscillation is suppressed, and a super luminescent light beam having a wide full width at half maximum spectrum is emitted from the light emitting end. The SLD 10 emits a 30mW super luminescent light beam having a central wavelength of 1.1$\mu$m and a full width at half maximum spectrum of 75nm.

The window region 111 of the SLD 10 is formed by p-type GaAs having a greater energy gap and a smaller refractive index than the InGaAs multiple quantum well active layer 105, with a lattice coefficient that lattice matches with GaAs within a range of $\pm 0.1\%$ and does not contain Al. Thereby, favorable crystal film qualities are realized in the window region layer 111 and the InGaAs multiple quantum well active layer 105. This extends the life of the element that emits a high output super luminescent light beam that has an undistorted sectional beam shape.

The SLD 10 is not exposed to an environment over 650° after formation of the InGaAs multiple quantum well active layer 105. Therefore, the InGaAs multiple quantum well active layer 105 does not deteriorate, and high output can be maintained for long periods of time.

Note that an SLD 10a having a p-type $In_{0.49}Ga_{0.51}P$ window region layer 111a may be employed instead of the SLD 10. P-type $In_{0.49}Ga_{0.51}P$ is also a semiconductor material which has a greater energy gap and a lower refractive index than the InGaAs multiple quantum well active layer 105 that lattice matches with GaAs within a range of $\pm 0.1\%$

and does not contain Al. The SLD 10a can also emit a high output super luminescent light beam having a central wavelength of 1.1μm and a full width at half maximum spectrum of 75nm, which has an undistorted sectional beam shape. In addition, the SLD 10a was continuously driven at room temperature to evaluate the element life thereof. Output levels fell to 90% of the initial output after approximately 5000 hours.

As a further alternative, an SLD 10b having an inner stripe structure as illustrated in Figure 11 may be employed instead of the SLD 10. As illustrated in Figure 11, the SLD 10b comprises: an optical waveguide path section 15; and a window section 16, which is provided at an end opposite a light emitting end of the optical waveguide path section 15. The optical waveguide path section 15 is of an inner stripe structure, wherein 3mm wide stripe structures formed on a p-type GaAs etching stop layer 108 constrict the flow of current. In addition, an n-type $(Al_{0.33}Ga_{0.67})_{0.5}As$ current blocking layer 119 (0.5μm thick, with a carrier density of $7.0\times10^{17}cm^{-3}$) is formed above a p-type GaAs window region layer 118 (0.5μm). The other structures are substantially the same as those of the SLD 10. An SLD 10c that comprises a p-type $In_{0.49}Ga_{0.51}P$ window region layer 118a may also be employed.

By employing the light source unit 210 comprising such SLD's, the reliability of the light source unit 210 is improved, and as a result, the reliability of the optical tomography apparatus 200 is also improved.

Note that the SLD 10 and the SLD 10b have SBR structures, while the SLD 10b and the SLD 10c have inner stripe structures. However, the structure of the SLD is not limited to these two examples. The SLD may have other index guide structures or gain guide structures.

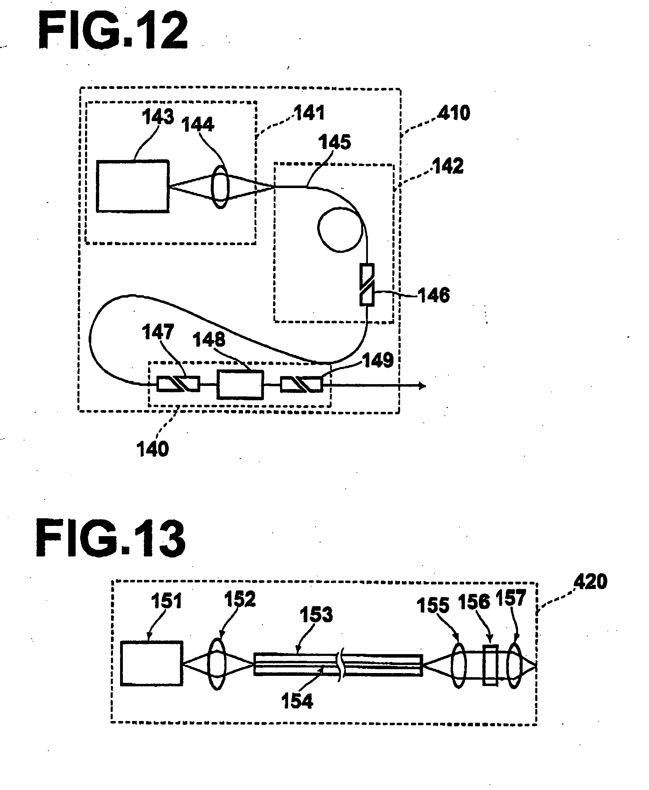

Note that a light source unit 410, such as that illustrated in Figure 12, may be considered for use instead of the light source unit 210. The light source unit 410 comprises: a pulse light source 141 that employs a mode locked solid state laser 143; a pulse compressing section 142; and a spectrum forming section 140. The pulse light source 141 comprises: the mode locked solid state laser 143; and a condensing lens 144, for guiding the pulse light beams emitted from the mode locked solid state laser 143 to the pulse compressing section 142. The pulse compressing section 142 comprises: a photonic crystal fiber 145 having negative dispersion properties; and an optical connector 146. The structural dispersion values of photonic crystal fibers are selectable. Therefore, negative dispersion properties can be realized with respect to a desired wavelength band. The spectrum forming section 140 comprises: an optical connector 147; a Gaussian distribution forming filter 148, for causing the spectrum shape of the wide band light beam output from the pulse compressing section 142 to become a Gaussian distribution; and an optical connector 149 for guiding the low coherence light beam, which has passed through the Gaussian distribution forming filter 148, to the fiber FB1. Note that the Gaussian distribution forming filter 148 forms the spectrum shape of the low coherence light beam such that the following conditions are satisfied.

$$\lambda c^2/\Delta\lambda \leq 23$$

$$\lambda c + (\Delta\lambda/2) \leq 1.2\mu m$$

$$\lambda c - (\Delta\lambda/2) \geq 0.98\mu m$$

As still another alternative, a light source unit 420 that comprises phosphor that contains near infrared fluorescent pigment, such as that illustrated in Figure 13, may be employed instead of the light source unit 210.

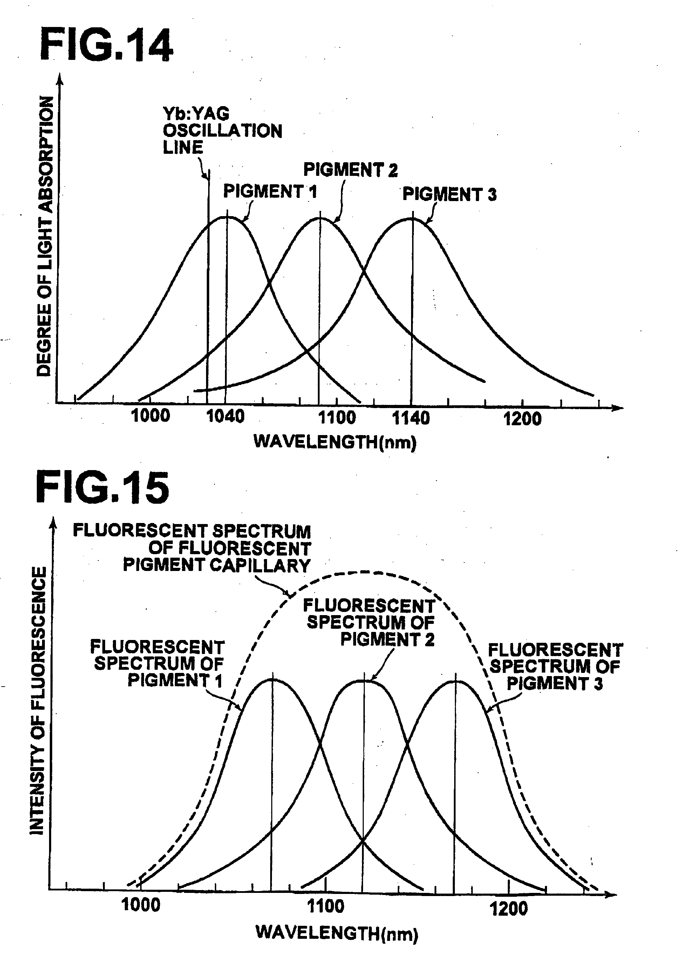

The light source unit 420 comprises: a capillary 153 that functions as the phosphor; a Yb:YAG laser 151 for exciting the capillary 153; an excitation light cutoff filter 156; and lenses 152, 155, and 157. Pigment, which is excitable by light having wavelengths within a range of 1.0 to 1.2μm, is sealed within the capillary 153 along with a solvent. Absorption spectra of pigments 1, 2, and 3 are illustrated in the graph of Figure 14. The pigments 1, 2, and 3 are excited by a light beam having a wavelength of 1.03μm emitted by the Yb: YAG laser. The fluorescence illustrated in Figure 15 can be obtained, by adjusting the concentration of each pigment such that the intensities of fluorescence are substantially equal among the three pigments. The sum of the fluorescence from each of the three pigments is a 1000nm to 1200nm spectrum as represented by the broken line in Figure 15. The wide band light beam generated in this manner passes through the lens 155, the excitation light cutoff filter 156 and the lens 157, and is guided to the fiber FB1 as the low coherence light beam La. Note that the excitation light cutoff filter 156 is provided to prevent the excitation light emitted from the Yb:YAG laser from entering the fiber FB1.

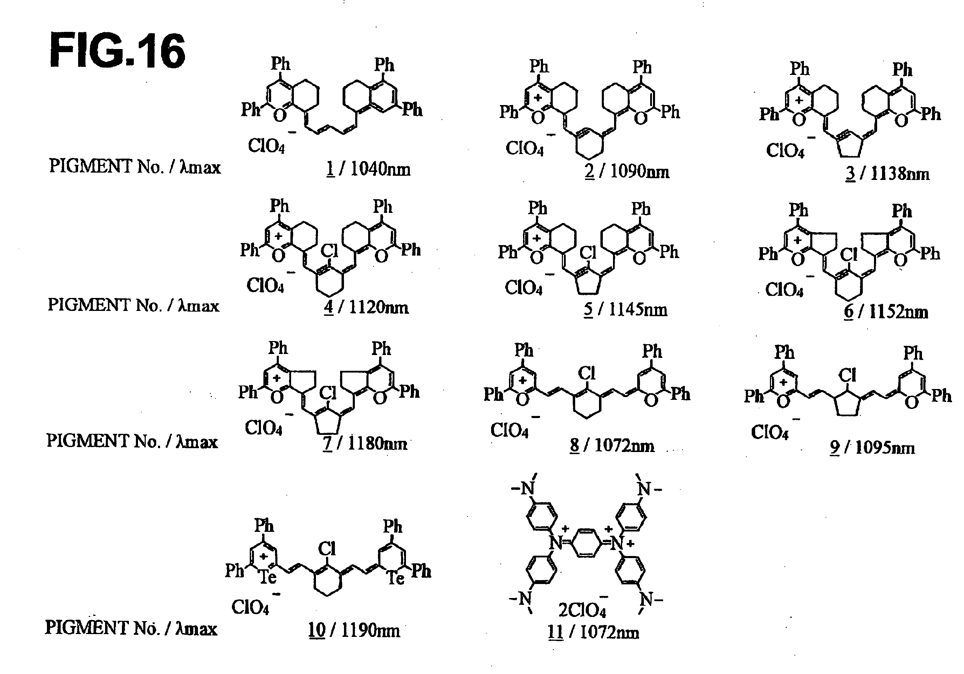

Pyrylium pigments, such as those illustrated in Figure 16, may be employed as the fluorescent pigment. Other appropriate lasers for emitting excitation light, such as an Nd:YLF laser that emits laser beams having a wavelength of 1.04μm and an Nd:YAG laser that emits laser beams having a wavelength of 1.064μm, can be selected depending on the combination of pigments.

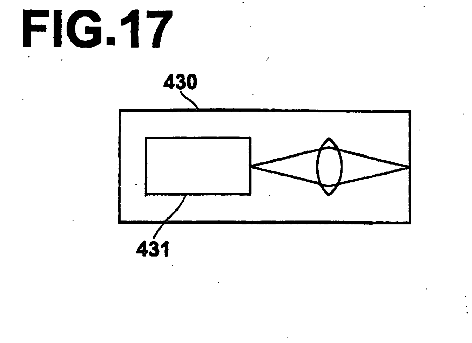

As a still further modification of the present embodiment, a light source unit 430, such as that illustrated in Figure 17, may be employed instead of the light source unit 210. The light source unit 430 comprises: a Yb type pulse laser, an Nd type pulse laser, or a Ti type pulse laser as a light source 431. A Yb:YAG laser, a Yb: glass laser, or a Yb type fiber laser may be utilized as the Yb type pulse laser. An Nd:YAG laser, an Nd: glass laser, or an Nd: fiber laser may be utilized as the Nd type pulse laser.

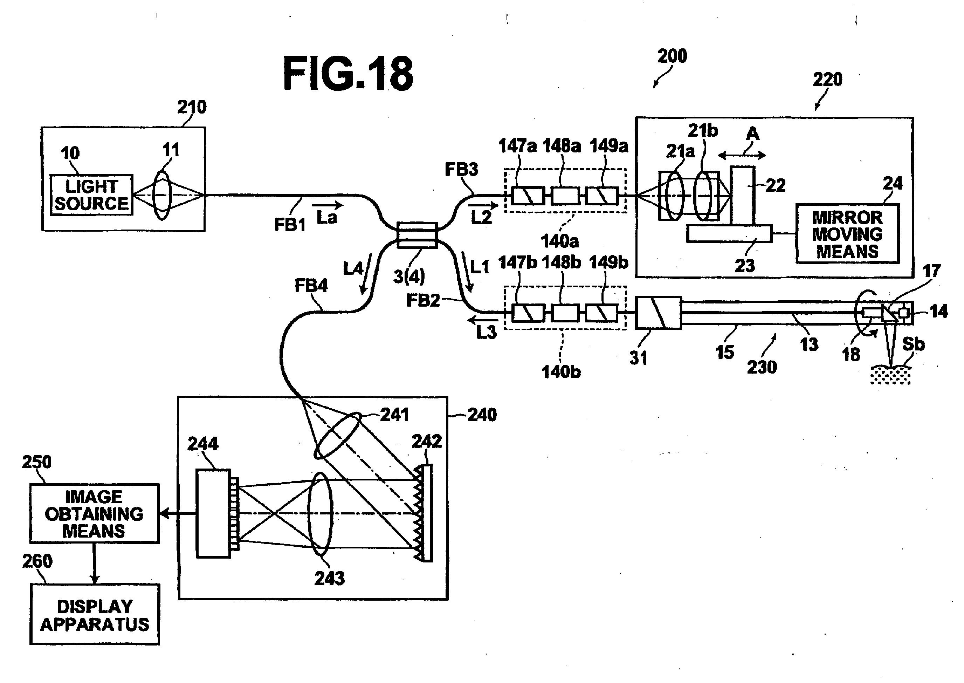

Note that the spectrum forming section 140 comprising the Gaussian distribution forming filter 148 may be provided in all of the aforementioned light source units 210, 420, and 430, in a manner similar to the light source unit 410. The spectrum forming section 140 may be provided at any position in the optical paths of the reference light beam L2 and the reflected light beam L3 prior to multiplexing. For example, a spectrum forming section 140a comprising: an optical connector 147a; a Gaussian distribution forming filter 148a; and an optical connector 149a may be provided along the optical path of the reference light beam L2, and a spectrum forming section 140b comprising: an optical connector 147b; a Gaussian distribution forming filter 148b; and an optical connector 149b may be provided along the optical path of the measuring light beam L1 (the reflected light beam L3), as illustrated in Figure 18.

In the case that the Gaussian distribution forming filter 148 is provided, the following conditions should be satisfied.

$$\lambda c^2 / \Delta\lambda \leq 23$$

$$\lambda c + (\Delta\lambda / 2) \leq 1.2\mu m$$

$$\lambda c - (\Delta\lambda / 2) \geq 0.98\mu m$$

That is, as long as the low coherence light beam satisfies the above conditions after passing through the Gaussian distribution forming filter 148, the light beam emitted from the SLD, the mode locked solid state laser, the phosphor that contains near infrared fluorescent pigment, or the pulse laser may have any central wavelength $\lambda c$ and any full width at half maximum spectrum $\Delta\lambda$. Note that in the case that the two Gaussian distribution forming filters 148a and 148b are provided, it is preferable that the filter properties are substantially uniform. However, the filter properties maybe different, as long as no adverse effects are exerted on measurement of the intensity of the coherent light beam.

**Claims**

1. An optical tomography apparatus (200), comprising:

  a light source (210, 410, 420, 430), for emitting a low coherence light beam (La);
  dividing means (3), for dividing the low coherence light beam (La) into a measuring light beam (L1) and a reference light beam (L2);
  an irradiating optical system, for irradiating the measuring light beam (L1) onto a measurement target (S);
  multiplexing means (4), for multiplexing a reflected light beam (L3), which is the measuring light beam (L1) reflected by the measurement target (S), and the reference light beam (L2), to obtain a coherent light beam (L4); and
  image obtaining means, for calculating the intensity of the reflected light beam (L3) at a plurality of depth positions of the measurement target (S), at which the optical path lengths of the measuring light beam (L1) and the reference light beam (L2) match, based on the properties of the multiplexed coherent light beam (L4), and for obtaining tomographic images of the measurement target (S), based on the intensities at each of the depth positions; the reference light beam (L2) and the reflected light beam (L3) having
  a central wavelength $\lambda c$ and a full width at half maximum spectrum $\Delta\lambda$
  satisfying the following conditions:

$$\lambda c^2 / \Delta\lambda \leq 23$$

$$\lambda c + (\Delta\lambda/2) \leq 1.2\mu m$$

$$\lambda c - (\Delta\lambda/2) \geq 0.98\mu m,$$

and
wherein
the light source (210) comprises a super luminescent diode (10, 10a, 10b, 10c), **characterized by**:
a GaAs substrate having a first conductivity;
an optical waveguide path constituted by an InGaAs active layer on the GaAs substrate; and
a window region layer having a greater energy gap and a smaller refractive index than the active layer and a second conductivity different from the first conductivity, constituted by a binary or ternary semiconductor material with a lattice coefficient that lattice matches with GaAs within a range of $\pm 0.1\%$ and does not contain Al, provided at a rear emitting facet of the optical waveguide path,
wherein:
the semiconductor material of the window region layer is one of GaAs and InGaP.

2. An optical tomography apparatus (200) as defined in Claim 1, wherein:

the central wavelength $\lambda c$ and the full width at half maximum spectrum $\Delta\lambda$ of the reference light beam (L2) and the reflected light beam (L3) satisfies the following condition:

$$\lambda c^2 / \Delta\lambda \leq 17.$$

3. An optical tomography apparatus (200) as defined in either of Claims 1 and 2, wherein:

the image obtaining means (250) is adapted to detect the intensity for each frequency of the coherent light beam (L4); and
to calculate the intensity of the reflected light beam (L3) at the plurality of depth positions of the measurement target (S), based on the detected intensities for each frequency of the coherent light beam (L4).

4. An optical tomography apparatus (200) as defined in any one of Claims 1 through 3, further comprising:

a Gaussian spectrum forming filter.


**Patentansprüche**

1. Optisches Tomographiegerät (200), umfassend:

eine Lichtquelle (210, 410, 420, 430) zum Emittieren eines Lichtstrahls (La) geringer Kohärenz;
eine Aufteileinrichtung (3) zum Aufteilen des Lichtstrahls (La) geringer Kohärenz in einen Messlichtstrahl (L1) und einen Referenzlichtstrahl (L2);
eine Aufstrahloptik zum Aufstrahlen des Messlichtstrahls (L1) auf ein Messobjekt (S);
eine Multiplexeinrichtung (4) zum Multiplexen eines reflektierten Lichtstrahls (L3), bei dem es sich um den von dem Messobjekt (S) reflektierten Messlichtstrahl (L1) handelt, und des Referenzlichtstrahls (L2), um einen kohärenten Lichtstrahl (L4) zu erhalten; und
eine Bildgewinnungseinrichtung zum Berechnen der Intensität des reflektierten Lichtstrahls (L3) an mehreren Tiefenstellen des Messobjekts (S), an welchen die optischen Weglängen des Messlichtstrahls (L1) und des Referenzlichtstrahls (L2) übereinstimmen, basierend auf den Eigenschaften des gemultiplexten kohärenten Lichtstrahls (L4), und zum Gewinnen von Tomographiebildern des Messobjekts (S), basierend auf den Intensitäten an jeder der Tiefenstellen; wobei der Referenzlichtstrahl (L2) und der reflektierte Lichtstrahl (L3) eine Mittenwellenlänge $\lambda c$ und
eine volle Breite bei halbem Maximum-Spektrum $\Delta\lambda$ aufweisen, die folgende Bedingungen erfüllen:

$$\lambda c^2/\Delta\lambda \leq 23$$

$$\lambda c + (\Delta\lambda/2) \leq 1{,}2 \ \mu m$$

$$\lambda c - (\Delta\lambda/2) \geq 0{,}98 \ \mu m,$$

und
wobei
die Lichtquelle (210) eine Superlumineszenzdiode (10, 10a, 10b, 10c) aufweist, **dadurch gekennzeichnet, dass** ein GaAs-Substrat mit einer ersten Leitfähigkeit vorgesehen ist; ein optischer Wellenleiterweg gebildet wird durch eine aktive InGaAs-Schicht auf dem GaAs-Substrat; und eine Fensterzonenschicht mit einer größeren Energielücke und einem kleineren Brechungsindex als die aktive Schicht und einer von der ersten Leitfähigkeit verschiedenen zweiten Leitfähigkeit vorgesehen ist, gebildet durch ein binäres oder ternäres Halbleitermaterial mit einem Gitterkoeffizienten, der mit dem GaAs innerhalb eines Bereichs von $\pm$ 0,1% gitterangepasst ist und kein Al enthält, vorgesehen in einer rückwärtigen Emissionsfacette des optischen Wellenleiterwegs, wobei das Halbleitermaterial der Fensterzonenschicht GaAs oder InGaP ist.

2. Optisches Tomographiegerät (200) nach Anspruch 1, bei dem:

die Mittenwellenlänge $\lambda c$ und die volle Breite bei halbem Maximum-Spektrum $\Delta\lambda$ des Referenzlichtstrahls (L2) und des reflektierten Lichtstrahls (L3) folgender Bedingung genügt:

$$\lambda c^2/\Delta\lambda \leq 17.$$

3. Optisches Tomographiegerät (200) nach Anspruch 1 oder 2, bei dem die Bildgewinnungseinrichtung (250) dazu ausgebildet ist, die Intensität für jede Frequenz des kohärenten Lichtstrahls (L4) nachzuweisen; und die Intensität des reflektierten Lichtstrahls (L3) an den mehreren Tiefenstellen des Messobjekts (S) basierend auf den nachgewiesenen Intensitäten für jede Frequenz des kohärenten Lichtstrahls (L4) zu berechnen.

4. Optisches Tomographiegerät (200) nach einem der Ansprüche 1 bis 3, weiterhin umfassend:

ein Gausspektrum-Filter.

**Revendications**

1. Appareil de tomographie optique (200), comprenant :

une source de lumière (210, 410, 420, 430), pour émettre un faisceau de lumière à faible cohérence (La) ; un moyen de division (3) pour diviser le faisceau de lumière à faible cohérence (La) en un faisceau de lumière de mesure (L1) et un faisceau de lumière de référence (L2) ; un système optique d'irradiation, pour irradier le faisceau de lumière de mesure (L1) sur une cible de mesure (S) ; un moyen de multiplexage (4) pour multiplexer un faisceau de lumière réfléchi (L3), qui est le faisceau de lumière de mesure (L1) réfléchi par la cible de mesure (S), et le faisceau de lumière de référence (L2), pour obtenir un faisceau de lumière cohérent (L4) ; et un moyen d'obtention d'image, pour calculer l'intensité du faisceau de lumière réfléchi (L3) à une pluralité de positions de profondeur de la cible de mesure (S), pour lesquelles les longueurs de trajet optique du faisceau de lumière de mesure (L1) et du faisceau de lumière de référence (L2) correspondent, en se basant sur les propriétés du faisceau de lumière cohérent multiplexé (L4), et pour obtenir des images tomographiques de la cible de mesure (S), en se basant sur les intensités dans chacune des positions de profondeur ;

le faisceau de lumière de référence (L2) et le faisceau de lumière réfléchi (L3) possédant une longueur d'onde centrale λc et une pleine largeur à la moitié du spectre maximum Δλ satisfaisant aux conditions suivantes :

$$\lambda c^2 / \Delta\lambda \leq 23$$

$$\lambda c + (\Delta\lambda/2) \leq 1,2 \ \mu m$$

$$\lambda c - (\Delta\lambda/2) \geq 0,98 \ \mu m,$$

et dans lequel

la source de lumière (210) comprend une diode superluminescente (10, 10a, 10b, 10c), **caractérisée par** un substrat en GaAs ayant une première conductivité ;

un trajet de guide d'onde optique constitué par une couche active d'InGaAs sur le substrat en GaAs ; et une couche de région de fenêtre ayant une plus grande bande interdite et un plus petit indice de réfraction que la couche active et une seconde conductivité différente de la première conductivité, constituée d'un matériau semi-conducteur binaire ou ternaire avec un coefficient de réseau qui correspond au GaAs du point de vue du réseau dans une plage de ± 0,1 % et ne contient pas d'Al, disposée sur une facette émettrice arrière du trajet de guide d'onde optique,

dans laquelle :

le matériau semiconducteur de la couche de région de fenêtre est un matériau parmi GaAs et InGaP.

2. Appareil de tomographie optique (200) selon la revendication 1, dans lequel :

la longueur d'onde centrale λc et la pleine largeur à la moitié du spectre maximum Δλ du faisceau de lumière de référence (L2) et du faisceau de lumière réfléchi (L3) satisfont à la condition suivante :

$$\lambda c^2 / \Delta\lambda \leq 17.$$

3. Appareil de tomographie optique (200) selon l'une ou l'autre des revendications 1 et 2, dans lequel :

le moyen d'obtention d'image (250) est adapté à détecter l'intensité pour chaque fréquence du faisceau de lumière cohérent (L4) ; et

à calculer l'intensité du faisceau de lumière réfléchi (L3) dans la pluralité de positions de profondeur de la cible de mesure (S), en se basant sur les intensités détectées pour chaque fréquence du faisceau de lumière cohérent (L4).

4. Appareil de tomographie optique (200) selon l'une quelconque des revendications 1 à 3, comprenant en outre :

un filtre de conformation de spectre gaussien.

# FIG.1A

# FIG.1B

## FIG.2

# FIG.3

ENDOSCOPIC REMOVAL

SURGICAL REMOVAL

m CANCER    m CANCER    sm CANCER

LAMINA MUSCULARIS MUCOSAE(MM)

LYMPH FOLLICLE

} MEMBRANA MUCOSA(m)LAYER

} SUBMUCOSAL(sm)LAYER

} TUNICA MUSCULARIS VENTRICULI

SEROUS MEMBRANE

# FIG.4

DISPERSION:D(fs·cm⁻¹nm⁻¹)

WAVELENGTH:λ(μm)

FIG.5

FIG.6A

FIG.6B

# FIG.7

# FIG.8A

| FWHM | 15.2 μm | 18.2 μm | 21.4 μm | 29.9 μm |

DISTANCE THROUGH WATER    0mm    2mm    4mm    10mm

# FIG.8B

| FWHM | 11.0 μm | 10.4 μm | 10.6 μm | 12.5 μm |

DISTANCE THROUGH WATER    0mm    2mm    4mm    10mm

# FIG.9

EP 1 705 476 B1

EP 1 705 476 B1

# FIG.10

10(10a)

115
114
110
113
109
105

108
107
112
111(111a)
106
104
103
102
101

12
13

# FIG.11

10b(10c)

115
120
117
116

105

108
107
119
118(118a)
106
104
103
102
101

15
16

22

# FIG.12

# FIG.13

# FIG.14

# FIG.15

# FIG.16

PIGMENT No. / λmax          $\underline{1}$ / 1040nm          $\underline{2}$ / 1090nm          $\underline{3}$ / 1138nm

PIGMENT No. / λmax          $\underline{4}$ / 1120nm          $\underline{5}$ / 1145nm          $\underline{6}$ / 1152nm

PIGMENT No. / λmax          $\underline{7}$ / 1180nm          $\underline{8}$ / 1072nm          $\underline{9}$ / 1095nm

PIGMENT No. / λmax          $\underline{10}$ / 1190nm          $\underline{11}$ / 1072nm

EP 1 705 476 B1

# FIG.17

**430**

**431**

# FIG.18

EP 1 705 476 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6377349 B

### Non-patent literature cited in the description

- **W. DREXLER et al.** In vivo ultrahigh-resolution optical coherence tomography. *Optics Letters,* 1999, vol. 24 (17), 1221-1223
- **I. HARTL et al.** Ultrahigh-resolution optical coherence tomography using continuum generation in an air-silica microstructure optical fiber. *Optics Letters,* vol. 26 (9), 608-610
- **Y. WANG et al.** Optimal wavelength for ultrahigh-resolution optical coherence tomography. *Optics Express,* vol. 11 (12), 1411-1417
- **SHIDLOVSKI V et al.** Superlumin-escent diodes for optical coherence tomography. *PROCEEDINGS OF THE SPIE - THE INTERNATIONAL SOCIETY FOR OPTICAL ENGINEERING SPIE-INT. SOC. OPT. ENG USA,* vol. 4648, 139-147
- **F. BUGGE et al.** *J. Crystal Growth,* vol. 272, 531-537